(19) Europäisches Patentamt
European Patent Office
Office européen des brevets

(11) Veröffentlichungsnummer: **0 653 425 A1**

# (12) EUROPÄISCHE PATENTANMELDUNG

(21) Anmeldenummer: **94117335.3**

(22) Anmeldetag: **03.11.94**

(51) Int. Cl.[6]: **C07D 471/04**, C07D 487/04, C07D 519/00, A61K 31/47, //(C07D471/04,221:00,209:00), (C07D487/04,209:00,209:00), (C07D519/00,471:00,471:00), (C07D519/00,487:00,471:00)

(30) Priorität: **16.11.93 DE 4339134**

(43) Veröffentlichungstag der Anmeldung: **17.05.95 Patentblatt 95/20**

(84) Benannte Vertragsstaaten: **AT BE CH DE DK ES FR GB GR IE IT LI LU MC NL PT SE**

(71) Anmelder: **BAYER AG**
**D-51368 Leverkusen (DE)**

(72) Erfinder: **Petersen, Uwe, Dr.**
**Auf dem Forst 4**
**D-51375 Leverkusen (DE)**
Erfinder: **Schenke, Thomas, Dr.**
**Mühlenstrasse 113**
**D-51469 Bergisch Gladbach (DE)**
Erfinder: **Böhm, Stefan, Dr.**
**Doerperhofstrasse 35**
**D-47800 Krefeld (DE)**
Erfinder: **Grosser, Rolf, Dr.**
**Gellertstrasse 9**
**D-51373 Leverkusen (DE)**
Erfinder: **Bremm, Klaus Dieter, Dr.**
**Eberhardstrasse 20**
**D-45661 Recklinghausen (DE)**
Erfinder: **Endermann, Rainer, Dr.**
**In den Birken 152a**
**D-42113 Wuppertal (DE)**
Erfinder: **Metzger, Karl-Georg, Dr.**
**Pahlkestrasse 75**
**D-42115 Wuppertal (DE)**

## (54) 1-(2-Fluorcyclopropyl)-chinolon- und naphthyridoncarbonsäure-Derivate, deren Herstellung und deren Verwendung in antibakteriellen Mitteln.

(57) Die Erfindung betrifft neue 1-(2-Fluorcyclopropyl)-chinolon- und naphthyridoncarbonsäure-Derivate, die in 7-Stellung durch einen (2,3,4,5,6,7-Hexahydro-1H-pyrrolo[3,4-c]pyridin-2-yl)- oder (1,2,3,4,5,6-Hexahydro-pyrrolo-[3,4-c]pyrrol-2-yl)-Rest substituiert sind, der Formel (I)

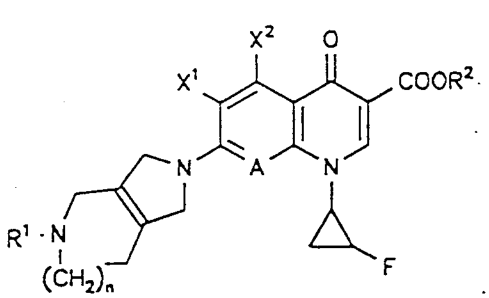

(I) ,

in welcher

$R^1$ für Wasserstoff, gegebenenfalls durch Hydroxy substituiertes $C_1$-$C_3$-Alkyl, $C_1$-$C_4$-Alkoxycarbonyl, ge-

EP 0 653 425 A1

gebenenfalls durch Halogen substituiertes Acetyl oder (5-Methyl-2-oxo-1,3-dioxol-4-yl)-methyl,

$R^2$ für Wasserstoff, gegebenenfalls durch Hydroxy, Methoxy, Amino, Methylamino oder Dimethylamino substituiertes Alkyl mit 1 bis 3 Kohlenstoffatomen, (5-Methyl-2-oxo-1,3-dioxol-4-yl)-methyl, Acetoxymethyl oder Pivaloyloxymethyl,

n für 0 oder 1,

$X^1$ für Halogen oder Nitro,

$X^2$ für Wasserstoff, Halogen, Amino oder Methyl,

A für N, C-H, C-F, C-Cl, C-Br, $C\text{-}CF_3$, $C\text{-}OCH_3$, $C\text{-}OCHF_2$, $C\text{-}CH_3$ oder C-C≡CH steht

und deren pharmazeutisch verwendbaren Hydrate und Säureadditionssalze sowie die Alkali-, Erdalkali-, Silber- und Guanidiniumsalze der zugrundeliegenden Carbonsäuren, Verfahren zu ihrer Herstellung sowie diese enthaltende antibakterielle Mittel.

Die Erfindung betrifft neue 1-(2-Fluorcyclopropyl)-chinolon- und naphthyridoncarbonsäure-Derivate, die in 7-Stellung durch einen (2,3,4,5,6,7-Hexahydro-1H-pyrrolo[3,4-c]pyridin-2-yl)- oder (1,2,3,4,5,6-Hexahydropyrrolo[3,4-c]pyrrol-2-yl)-Rest substituiert sind, ihre Salze, Verfahren zu ihrer Herstellung sowie diese enthaltende antibakterielle Mittel.

Es sind bereits aus den Patentaumeldungen EP 424 850 (Korea Research Institute), EP 424 851 (Korea Research Institute) und EP 520 277 (Bayer) Chinoloncarbonsäuren bekanntgeworden, die einen (2,3,4,5,6,7-Hexahydro-1H-pyrrolo[3,4-c]pyridin-2-yl)-Rest in der 7-Stellung tragen. Andererseits sind aus WO 9221659 (Daiichi) 1-(2-Fluorcyclopropyl)-chinoloncarbonsäuren bekanntgeworden.

Es wurde jetzt gefunden, daß die Verbindungen der Formel (I)

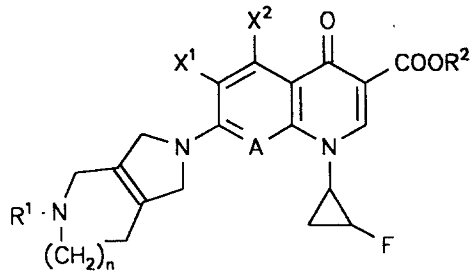

(I) ,

in welcher

- $R^1$ für Wasserstoff, gegebenenfalls durch Hydroxy substituiertes $C_1$-$C_3$-Alkyl, $C_1$-$C_4$-Alkoxycarbonyl, gegebenenfalls durch Halogen substituiertes Acetyl oder (5-Methyl-2-oxo-1,3-dioxol-4yl)-methyl,
- $R^2$ für Wasserstoff, gegebenenfalls durch Hydroxy, Methoxy, Amino, Methylamino oder Dimethylamino substituiertes Alkyl mit 1 bis 3 Kohlenstoffatomen, (5-Methyl-2-oxo-1,3-dioxol-4-yl)-methyl, Acetoxymethyl oder Pivaloyloxymethyl,
- n für 0 oder 1,
- $X^1$ für Halogen oder Nitro,
- $X^2$ für Wasserstoff, Halogen, Amino oder Methyl,
- A für N, C-H, C-F, C-Cl, C-Br, C-$CF_3$, C-$OCH_3$, C-$OCHF_2$, C-$CH_3$ oder C-C≡CH steht

und deren pharmazeutisch verwendbaren Hydrate und Säureadditionssalze sowie die Alkali-, Erdalkali-, Silber- und Guanidiniumsalze der zugrundeliegenden Carbonsäuren bei guter Verträglichkeit eine hohe antibakterielle Wirkung insbesondere gegenüber grampositiven Bakterien aufweisen.

Bevorzugt sind die Verbindungen der Formel (I),
in welcher

- $R^1$ für Wasserstoff, Methyl, Ethyl, t-Butoxycarbonyl, Acetyl, Trifluoracetyl oder Trichloracetyl
- $R^2$ für Wasserstoff,
- n für 0 oder 1,
- $X^1$ für Chlor oder Fluor,
- $X^2$ für Wasserstoff, Fluor, Amino oder Methyl,
- A für N, C-H, C-F, C-Cl, C-Br, C-$CF_3$, C-$OCH_3$, C-$OCHF_2$, C-$CH_3$ oder C-C≡CH steht

und deren pharmazeutisch verwendbaren Hydrate und Säureadditionssalze sowie die Alkali-, Erdalkali-, Silber- und Guanidiniumsalze der zugrundeliegenden Carbonsäuren.

Besonders bevorzugt sind die Verbindungen der Formel (I),
in welcher

- $R^1$ für Wasserstoff oder Methyl,
- $R^2$ für Wasserstoff,
- n für 1,
- $X^1$ für Fluor,
- $X^2$ für Wasserstoff, Fluor oder Amino,
- A für N, C-H, C-F, C-Cl, C-Br, C-$OCH_3$ steht

und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn man Verbindungen der Formel (II)

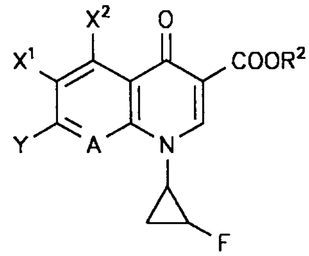

(II) ,

in welcher

$R^2$, $X^1$, $X^2$ und A die oben angegebene Bedeutung haben und

Y für Halogen, inbesondere für Fluor oder Chlor steht,

mit Verbindungen der Formel (III)

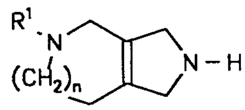

(III) ,

in welcher

$R^1$ und n die oben angegebenen Bedeutungen haben,

gegebenenfalls in Gegenwart von Säurefängern umsetzt und gegebenenfalls vorhandene Schutzgruppen abspaltet.

Verwendet man beispielsweise 6,7-Difluor-1-(cis-2-fluor-cyclopropyl)-1,4-dihydro-8-methoxy-4-oxo-3-chinolincarbonsäure und 5-tert. Butoxycarbonyl-2,3,4,5,6,7-hexahydro-1H-pyrrolo[3,4-c]pyridin als Ausgangsstoffe, so kann der Reaktionsverlauf durch das folgende Formelschema wiedergegeben werden:

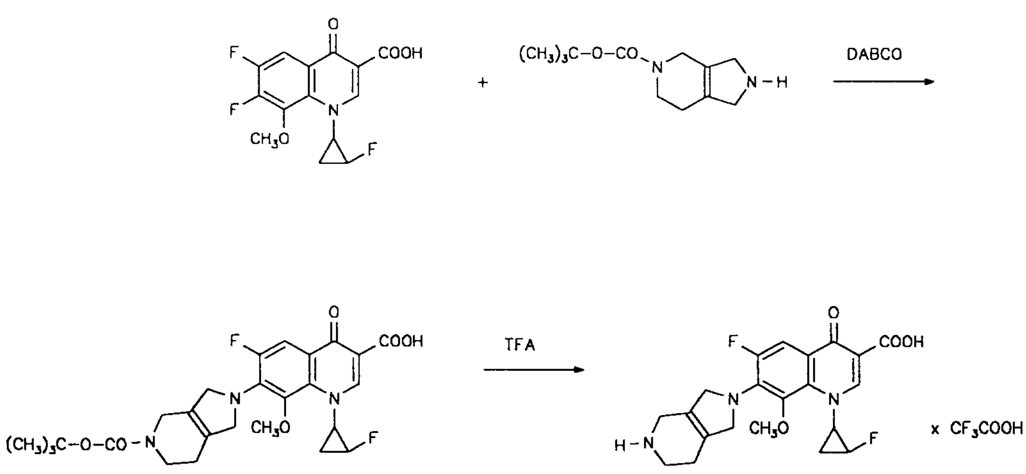


DABCO = 1,4-Diazabicyclo[2.2.2]octan; TFA = Trifluoressigsäure

Die als Ausgangsverbindungen verwendeten Verbindungen der Formel (II) sind bekannt oder können nach bekannten Methoden hergestellt werden. Sie können sowohl als racemische als auch als enantiomerenreine Verbindungen eingesetzt werden. Als Beispiele seien genannt:

8-Brom-6,7-difluor-1-(cis-2-fluor-cyclopropyl)-1,4-dihydro-4-oxo-3-chinolincarbonsäure,
8-Chlor-6,7-difluor-1-(cis-2-fluor-cyclopropyl)-1,4-dihydro-4-oxo-3-chinolincarbonsäure,
6,7-Difluor-1-(cis-2-fluor-cyclopropyl)-1,4-dihydro-4-oxo-3-chinolincarbonsäure,
6,7,8-Trifluor-1-(cis-2-fluorcyclopropyl)-1,4-dihydro-4-oxo-3-chinolincarbonsäure,
5,6,7,8-Tetrafluor-1-(cis-2-fluor-cyclopropyl)-1,4-dihydro-4-oxo-3-chinolincarbonsäure,
6,7-Difluor-1-(cis-2-fluor-cyclopropyl)-1,4-dihydro-8-methyl-4-oxo-3-chinolincarbonsäure,
8-Ethinyl-6,7-difluor-1-(cis-2-fluor-cyclopropyl)-1,4-dihydro-4-oxo-3-chinolincarbonsäure,
6,7-Difluor-1-(cis-2-fluor-cyclopropyl)-1,4-dihydro-4-oxo-8-trifluormethyl-3-chinolincarbonsäure,
6,7-Difluor-1-(cis-2-fluor-cyclopropyl)-8-difluormethoxy-1,4-dihydro-4-oxo-3-chinolincarbonsäure,

6,7-Difluor-1-(cis-2-fluor-cyclopropyl)-1,4-dihydro-8-methoxy-4-oxo-3-chinolincarbonsäure,
6,7-Difluor-1-(cis-2-fluor-cyclopropyl)-1,4-dihydro-5-methyl-4-oxo-3-chinolincarbonsäure,
5-Amino-6,7,8-trifluor-1-(cis-2-fluor-cyclopropyl)-1,4-dihydro-4-oxo-3-chinolincarbonsäure,
8-Brom-6,7-difluor-1-(trans-2-fluor-cyclopropyl)-1,4-dihydro-4-oxo-3-chinolincarbonsäure,
8-Chlor-6,7-difluor-1-(trans-2-fluor-cyclopropyl)-1,4-dihydro-4-oxo-3-chinolincarbonsäure,
6,7-Difluor-1-(trans-2-fluor-cyclopropyl)-1,4-dihydro-4-oxo-3-chinolincarbonsäure,
6,7,8-Trifluor-1-(trans-2-fluor-cyclopropyl)-1,4-dihydro-4-oxo-3-chinolincarbonsäure,
5,6,7,8-Tetrafluor-1-(trans-2-fluor-cyclopropyl)-1,4-dihydro-4-oxo-3-chinolincarbonsäure,
6,7-Difluor-1-(trans-2-fluor-cyclopropyl)-1,4-dihydro-8-methyl-4-oxo-3-chinolincarbonsäure,
8-Ethinyl-6,7-difluor-1-(trans-2-fluor-cyclopropyl)-1,4-dihydro-4-oxo-3-chinolincarbonsäure,
6,7-Difluor-1-(trans-2-fluor-cyclopropyl)-1,4-dihydro-4-oxo-8-trifluormethyl-3-chinolincarbonsäure,
6,7-Difluor-1-(trans-2-fluor-cyclopropyl)-8-difluormethoxy-1,4-dihydro-4-oxo-3-chinolincarbonsäure,
6,7-Difluor-1-(trans-2-fluor-cyclopropyl)-1,4-dihydro-8-methoxy-4-oxo-3-chinolincarbonsäure,
6,7-Difluor-1-(trans-2-fluor-cyclopropyl)-1,4-dihydro-5-methyl-4-oxo-3-chinolincarbonsäure,
5-Amino-6,7,8-Trifluor-1-(trans-2-fluor-cyclopropyl)-1,4-dihydro-4-oxo-3-chinolincarbonsäure,
Zur Herstellung der enantiomerenreinen Ausgangsverbindungen der Formel (II) kann man von enantiomerenreinen 2-Fluorcyclopropylaminen ausgehen. Man kann aber auch die racemischen Verbindungen der Formel (II) mit enantiomerenreinen Basen zu einem Gemisch der diastereomeren Salze umsetzen, die sich durch fraktionierte Kristallisation zu den diastereomerenreinen Salzen trennen lassen, aus denen man durch Behandlung mit geeigneten Säuren, zum Beispiel Mineralsäuren wie Salzsäure oder Schwefelsäure, die enantiomerenreinen Verbindungen der Formel (II) freisetzen kann.

Vorteilhaft ist aber auch eine chromatographische Trennung der racemischen Verbindungen der Formel (II) an chiralen Trennmaterialien möglich. Hierzu gehören bevorzugt optisch aktive Polymere von optisch aktiven (Meth)-acrylsäure-Derivaten. Besonders bevorzugt sind hier Polymerisate von optisch aktiven N-(Meth)-acryloyl-aminosäure-Derivaten, wie sie in der Europäischen Patentanmeldung 379 917 beschrieben sind. Ganz besonders bevorzugt seien hier Polymerisate aus folgenden optisch aktiven N-Acryloyl-aminosäureestern genannt: N-Acryloyl-L- beziehungsweise D-aminosäure-menthylester, wobei als Aminosäure zum Beispiel Leucin, Alanin, Phenylalanin, Valin oder sonstige Aminosäuren in Frage kommen.

Als Fließmittel für die Trennung des Racemats werden übliche organische Lösemittel beziehungsweise Lösemittelgemische verwendet, die das als Adsorbens eingesetzte Polymerisat anquellen und das zu trennende Racemat lösen. Beispielsweise seien genannt: Kohlenwasserstoffe wie Benzol, Toluol oder Xylol, Ether wie Diethylether, Dioxan oder Tetrahydrofuran, Halogenkohlenwasserstoffe wie Di- oder Trichlormethan, Aceton, Acetonitril oder Essigester oder aber Gemische der genannten Lösemittel. Als besonders geeignet haben sich Mischungen aus Toluol und Tetrahydrofuran sowie aus Toluol und Dioxan erwiesen.

Die als Ausgangsverbindungen benötigten bicyclischen Amine der Formel (III) sind teilweise bekannt. Als Beispiele seien genannt:
2,3,4,5,6,7-Hexahydro-1H-pyrrolo[3,4-c]pyridin,
5-Ethyl-2,3,4,5,6,7-hexahydro-1H-pyrrolo[3,4-c]pyridin,
5-Methyl-2,3,4,5,6,7-hexahydro-1H-pyrrolo[3,4-c]pyridin,
5-(2-Hydroxyethyl)-2,3,4,5,6,7-hexahydro-1H-pyrrolo[3,4-c]pyridin,
5-tert.-Butoxycarbonyl-2,3,4,5,6,7-hexahydro-1H-pyrrolo[3,4-c]pyridin,
5-Trifluoracetyl-2,3,4,5,6,7-hexahydro-1H-pyrrolo[3,4-c]pyridin,
Hexahydro-pyrrolo[3,4-c]pyrrol,
2-Methyl-hexahydro-pyrrolo[3,4-c]pyrrol,
2-Ethyl-hexahydro-pyrrolo[3,4-c]pyrrol.

Die Umsetzung von (II) mit (III), bei der die Verbindungen (III) auch in Form ihrer Salze, wie zum Beispiel der Hydrochloride, eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanal, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200 °C, vorzugsweise zwischen 80 und 160 °C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 5 Mol der Verbindung (III) ein.

Freie Aminogruppen können während der Umsetzung durch eine geeignete Aminoschutzgruppe, wie zum Beispiel durch den tert.-Butoxycarbonylrest, geschützt und nach Beendigung der Reaktion wieder freigesetzt werden.

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20 bis 180 °C, vorzugsweise etwa 60 bis 120 °C, umgesetzt. Das entstehende Reaktionswasser kann auch durch azeotrope Destillation mit Chloroform, Tetrachlormethan oder Toluol entfernt werden.

Die Herstellung von Estern gelingt auch vorteilhaft durch Erhitzen der zugrundeliegenden Säure mit Dimethylformamiddialkylacetal in einem Lösungsmittel wie Dimethylformamid.

Die als Prodrug verwendeten (5-Methyl-2-oxo-1,3-dioxol-4-yl-methyl)-ester werden durch Umsetzung eines Alkalisalzes der zugrundliegenden Carbonsäure, die gegebenenfalls am N-Atom durch eine Schutzgruppe wie den tert.-Butoxycarbonylrestgeschützt sein kann, mit 4-Brommethyl- oder 4-Chlormethyl-5-methyl-1,3-dioxol-2-on in einem Lösungsmittel wie Dimethylformamid, Dimethylacetamid, N-Methyl-pyrrolidon, Dimethylsulfoxid oder Tetramethylharnstoff bei Temperaturen von etwa 0 bis 100 °C, vorzugsweise 0 bis 50 °C, erhalten.

Die Herstellung der Säureadditionssalze der erfindungsgemäßen Verbindungen erfolgt in üblicher Weise zum Beispiel durch Lösen in überschüssiger wäßriger Säure und Ausfällen des Salzes mit einem mit Wasser mischbaren Lösungsmittel wie Methanol, Ethanol, Aceton oder Acetonitril. Man kann auch äquivalente Menge Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, 2-Hydroxyglutarsäure, Methansulfonsäure, 4-Toluolsulfonsäure, Galacturonsäure, Glucuronsäure, 5-Oxotetrahydrofuran-2-carbonsäure, Embonsäure, Glutaminsäure oder Asparaginsäure zu verstehen.

Die Alkali- und Erdalkalisalze der erfindungsgemäßen Carbonsäuren werden beispielsweise durch Lösen des Betains in überschüssiger Alkali- oder Erdalkalilauge, Filtration vom ungelösten Betain und Eindampfen bis zur Trockne erhalten. Pharmazeutisch geeignet sind Natrium, Kalium- und Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die in der folgenden Tabelle aufgeführten Wirkstoffe hergestellt werden, die sowohl als Racemate oder als enantiomerenreine Verbindungen oder gegebenenfalls auch als Diastereomerengemische oder als diastereomerenreine Verbindungen vorliegen können:

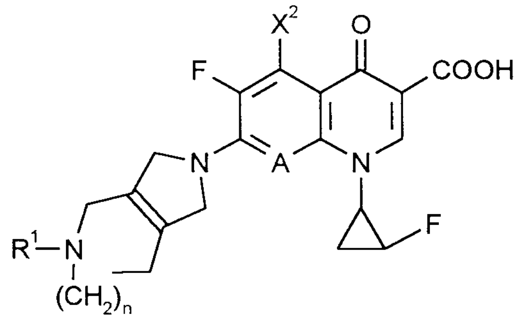

| $R^1$ | n | $X^2$ | A |
|---|---|---|---|
| $CH_3$ | 1 | $NH_2$ | N |
| $CH_3$ | 1 | H | C-$OCHF_2$ |
| $CH_3$ | 1 | H | C-Br |
| $CH_3$ | 1 | H | C-$CF_3$ |
| $CH_3$ | 1 | H | C-$OCH_3$ |
| $CH_3$ | 1 | H | C-$CH_3$ |
| $CH_3$ | 1 | H | C-C≡CH |
| $CH_3$ | 1 | F | C-F |
| $CH_3$ | 1 | $NH_2$ | C-F |
| $CH_3$ | 1 | $CH_3$ | C-F |
| $CH_3$ | 1 | $CH_3$ | H |
| H | 1 | H | N |
| H | 1 | H | C-H |
| H | 1 | H | C-F |
| H | 1 | H | C-Cl |
| H | 1 | H | C-CBr |

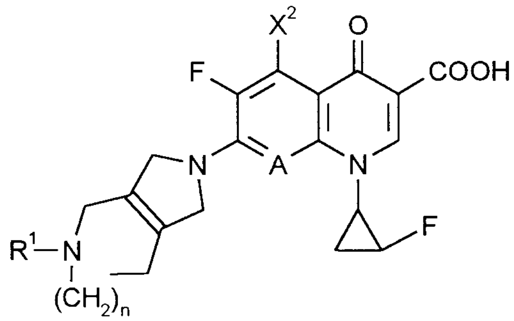

| $R^1$ | n | $X^2$ | A |
|---|---|---|---|
| H | 1 | H | C-$CF_3$ |
| H | 1 | H | C-$OCH_3$ |
| H | 1 | H | C-$OCHF_2$ |
| H | 1 | H | C-$CH_3$ |
| H | 1 | H | C-C≡CH |
| H | 1 | F | C-F |
| H | 1 | $NH_2$ | C-F |
| H | 1 | $CH_3$ | C-F |
| H | 1 | $CH_3$ | C-H |
| H | 0 | H | N |
| H | 0 | H | C-H |
| H | 0 | H | C-F |
| H | 0 | H | C-Cl |
| H | 0 | H | C-Br |
| H | 0 | H | C-$OCH_3$ |
| H | 0 | H | C-$OCHF_2$ |

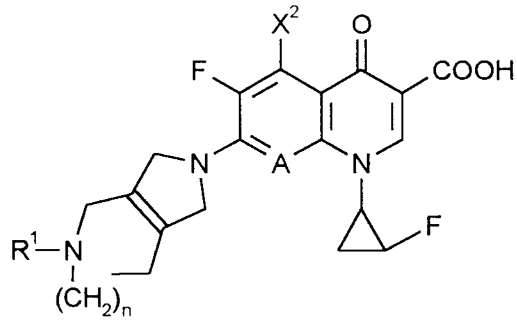

| $R^1$ | n | $X^2$ | A |
|---|---|---|---|
| H | 0 | H | $C-CH_3$ |
| H | 0 | H | C-C≡CH |
| $CH_3$ | 0 | H | N |
| $CH_3$ | 0 | H | C-H |
| $CH_3$ | 0 | H | C-F |
| $CH_3$ | 0 | H | C-Cl |
| $CH_3$ | 0 | H | C-Br |
| $CH_3$ | 0 | H | $C-OCH_3$ |
| $CH_3$ | 0 | H | $C-OCHF_2$ |
| $CH_3$ | 0 | H | $C-CH_3$ |
| $CH_3$ | 0 | H | C-C≡CH |
| $HO-CH_2CH_2$ | 0 | H | C-F |
| $HO-CH_2CH_2$ | 0 | H | C-Cl |
| $HO-CH_2CH_2$ | 0 | H | C-H |
| $HO-CH_2CH_2$ | 1 | H | C-F |
| $HO-CH_2CH_2$ | 1 | H | C-Cl |

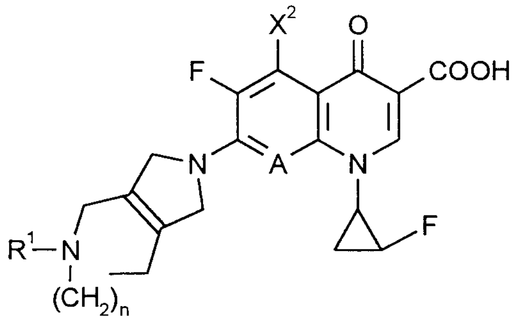

| $R^1$ | $R^2$ | n | $X^1$ | $X^2$ | A |
|---|---|---|---|---|---|
| H | H | 0 | Cl | H | C-H |
| H | H | 0 | Cl | H | C-F |
| H | H | 1 | Cl | H | N |
| H | $C_2H_5$ | 0 | F | H | C-F |
| H | $C_2H_5$ | 1 | F | H | C-F |
| H | $C_2H_5$ | 1 | F | H | $C\text{-}OCH_3$ |
| H | H | 0 | $NO_2$ | H | C-F |
| H | $C_2H_5$ | 0 | F | $NH_2$ | C-F |
| H | $HO\text{-}CH_2CH_2$ | 0 | F | H | C-F |
| H | H | 0 | F | F | C-F |
| $CH_3$ | $(CH_3)_2N\text{-}CH_2CH_2$ | 1 | F | H | C-F |
| $CH_3$ | H | 1 | Cl | H | C-H |
| $CH_3$ | H | 0 | Cl | H | C-F |
| $CH_3$ | $C_2H_5$ | 0 | F | F | $C\text{-}OCH_3$ |
| $CH_3$ | $C_2H_5$ | 0 | F | H | C-Cl |
| $CH_3$ | $CH_3$ | 0 | F | H | $C\text{-}OCH_3$ |

Die erfindungsgemäßen Verbindungen wirken stark antibiotisch und zeigen bei geringer Toxizität ein breites antibakterielles Spektrum gegenüber grampositiven und gramnegativen Keimen, vor allem auch gegnüber solchen, die gegen verschiedene Antibiotika, wie zum Beispiel Penicilline, Cephalosporine, Aminoglykoside, Sulfonamide, Tetracycline sowie gegen handelsübliche Chinolone, resistent sind.

Diese wertvollen Eigenschaften ermöglichen ihre Verwendung als chemotherapeutische Wirkstoffe in der Medizin sowie als Stoffe zur Konservierung von anorganischen und organischen Materialien, zum

Beispiel von Polymeren, Schmiermitteln, Farben, Fasern, Leder, Papier und Holz, von Lebensmitteln und von Wasser.

Die erfindungsgemäßen Verbindungen sind gegen ein sehr breites Sprektrum von Mikroorganismen wirksam. Mit ihrer Hilfe können gramnegative und grampostive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch eine verstärkte Wirkung auf ruhende Keime aus. Bei ruhenden Bakterien, also Bakterien, die kein nachweisbares Wachstum zeigen, wirken die Verbindungen stark bakterizid. Dies bezieht sich nicht nur auf die einzusetzende Menge, sondern auch auf die Geschwindigkeit der Abtötung. Solche Ergebnisse konnten bei grampositiven und gramnegativen Bakterien, insbesondere bei Staphylococcus aureus, Pseudomonas aeruginosa, Enterococcus faecalis und Escherichia coli beobachtet werden.

Besonders wirksam sind die erfindungsgemäßen Verbindungen gegen typische und atypische Mykobakterien und Helicobacter pylori sowie gegen bakterienähnliche Mikroorganismen, wie zum Beispiel Mykoplasmen und Rickettsien. Sie sind daher besonders gut in der Human- und Tiermedizin zur Prophylaxe und Chemotherapie von lokalen und systemischen Infektionen geeignet, die durch diese Erreger hervorgerufen werden.

Die Verbindungen eignen sich ferner besonders gut zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die erfindungsgemäßen Verbindungen können auch mit $\beta$-Lactamderivaten wie zum Beispiel Cephalosporinen oder Penemen über kovalente Bindungen zu sogenannten Dual-Action-Derivaten verknüpft werden.

Die minimalen Hemmkonzentrationen (MHK) wurden durch ein Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt circa $10^4$ koloniebildende Partikel enthielt. Die beimpften Agarplatten wurden bei 37 °C bebrütet und das Keimwachstum nach circa 20 Stunden abgelesen. Der MHK-Wert ($\mu$g/ml) gibt die niedrigste Wirkstoffkonzentration an, bei der mit bloßem Auge kein Wachtum zu erkennen war.

In Tabelle 1 sind die MHK-Werte einiger der erfindungsgemäßen Verbindungen im Vergleich zu 1-Cyclopropyl-6,8-difluor-1,4-dihydro-7-(5-methyl-2,3,4,5,6,7-hexahydro-1H-pyrrolo[3,4-c]pyridin-2-yl)-4-oxo-3-chinolincarbonsäure-Hydrochlorid (Ref. 1: Beispiel 7 B aus der Europäischen Patentanmeldung 520 277) sowie 8-Chlor-1-cyclopropyl-6-fluor-1,4-dihydro-7-(5-methyl-2,3,4,5,6,7-hexahydro-1H-pyrrolo[3,4-c]pyridin-2-yl)-4-oxo-3-chinolincarbonsäure (Ref. 2: Beispiel 8 aus der Europäischen Patentanmeldung 520 277) aufgeführt:

Tabelle 1: MHK-Werte (µg/ml)

| Species | Strain | Beipiel 1 | 2 | 3 | 4 | Ref. 1 | Ref. 2 |
|---|---|---|---|---|---|---|---|
| Escherichia coli | Neumann | ≤0,015 | 0,03 | ≤0,015 | ≤0,015 | ≤0,015 | 0,03 |
| Klebsiella sp. | 8085 | 0,06 | 0,06 | 0,06 | 0,06 | 0,06 | 0,06 |
| Morganella morganii | 932 | 0,03 | 0,06 | 0,06 | 0,06 | 0,03 | 0,06 |
| Providencia sp. | 12012 | 0,06 | 0,06 | 0,06 | 0,06 | 0,125 | 0,125 |
| Micrococcus luteus | 9341 | 0,25 | 0,25 | 0,125 | 0,125 | 0,5 | 0,25 |
| Staphylococcus aureus | ICB 25701 | 0,5 | 0,5 | 0,5 | 0,25 | 1 | 0,5 |
| | 133 | 0,06 | 0,06 | 0,06 | ≤0,015 | 0,6 | 0,03 |
| Enterococcus faecalis | 27101 | 0,06 | 0,06 | 0,06 | 0,06 | 0,125 | 0,06 |

Die erfindungsgemäßen Verbindungen zeichnen sich inbesondere dadurch aus, daß sie im Vergleich zu den Verbindungen nach dem Stand der Technik sowohl eine bessere akute Verträglichkeit besitzen als auch geringere Interaktionen mit Säugetier-DNA aufweisen. Die Ergebnisse sind in der Tabelle 2 wiedergegeben. Die hierin aufgeführten $LD_{50}$-Werte wurden nach intravenöser Verabreichung der Substanzen an CF1-Mäusen bestimmt. Unter $ID_{50}$ versteht man die Konzentration eines Stoffes, bei der die DNA-Synthese

in Zellen aus Ovarien des chinesischen Hamsters (CHO-KI) um 50 % gehemmt wird. Dieser Wert wird nach Inkubation der entsprechenden Substanzen in abfallenden Verdünnungsstufen über definierte Zeiträume bestimmt. Dazu wird mittels fluorophotometrischer Methoden die DNA-Synthese in CHO-KI-Zellen im Vergleich zu Kontrollen ermittelt.

Tabelle 2

| Verträglichkeitsparameter | | | | | |
|---|---|---|---|---|---|
| | Beispiel | | | | |
| | 1 | 2 | 3 | Ref. 1 | Ref. 2 |
| $LD_{50}$ (mg/kg) | 150 | 200 | 50 | 50 | 50 |
| $ID_{50}$ (µg/ml) | 35 | 40 | 40 | 1 | 16 |

Herstellung der Zwischenprodukte

Beispiel Z 1

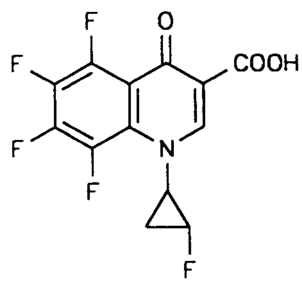

A. Zu einer Suspension von 2,5 g (22 mmol) racemischem trans-2-Fluorcyclopropylamin-Hydrochlorid in 10 ml Essigsäure gibt man unter Eiskühlung 1,3 g (11,6 mmol) 1,4-Diazabicyclo[2.2.2]octan und tropft innerhalb 10 Minuten eine Lösung aus 7 g (ca. 20 mmol) 3-Dimethylamino-2-(pentafluorbenzoyl)-acrylsäureethylester in 8 ml Essigsäure zu. Man läßt ohne Kühlung 4 Stunden nachrühren, engt bei 70 °C/15 mbar ein, nimmt den Rückstand in 25 ml Dichlormethan auf, wäscht mit Wasser, trocknet mit Natriumsulfat, engt erneut ein und reinigt durch Chromatographie an 200 g Kieselgel mit Dichlormethan als Laufmittel. Man isoliert ein Öl, das langsam durchkristallisiert.
Ausbeute: 2,1 g (27,5 % der Theorie) 3-(trans-2-Fluor-cyclopropylamino)-2-(pentafluorbenzoyl)-acrylsäureethylester,
Schmelzpunkt: 103-105 °C.

B. 2 g (5,4 mmol) 3-(trans-2-Fluor-cyclopropylamino)-2-(pentafluorbenzoyl)-acrylsäureethylester werden in 10 ml Dimethylformamid mit 250 mg (6 mmol) Natriumfluorid versetzt und 2 Stunden unter Rückfluß erhitzt. Nach dem Abkühlen wird die Suspension in 50 ml Eiswasser eingetragen, der ungelöste Rückstand abgesaugt, mit Wasser gewaschen und bei 80 °C im Hochvakuum getrocknet.
Ausbeute: 1,4 g (74 % der Theorie) 5,6,7,8-Tetrafluor-1-(trans-2-fluor-cyclopropyl)-1,4-dihydro-4-oxo-3-chinolincarbonsäureethylester,
Schmelzpunkt: 135-138 °C (unter Zersetzung).

C. 1,35 g (3,9 mmol)5,6,7,8-Tetrafluor-1-(trans-2-fluorcyclopropyl)-1,4-dihydro-4-oxo-3-chinolincarbonsäureethylester werden in einer Mischung aus 6,3 ml Essigsäure, 4,2 ml Wasser und 0,64 ml Konzentrierter Schwefelsäure 2 Stunden unter Rückfluß erhitzt. Nach dem Abkühlen wird in 40 ml Eiswasser eingetragen, der ungelöste Niederschlag abfiltriert, mit Wasser gewaschen und bei 110 °C im Hochvakuum getrocknet.
Ausbeute: 0,9 g (72 % der Theorie) 5,6,7,8-Tetrafluor-1-(trans-2-fluor-cyclopropyl)-1,4-dihydro-4-oxo-3-chinolincarbonsäure,
Schmelzpunkt: 184-186 °C (unter Zersetzung).

Analog wird 5,6,7,8-Tetrafluor-1-(cis-2-fluor-cyclopropyl)-1,4-dihydro-4-oxo-3-chinolincarbonsäure hergestellt.

Beispiel Z 2

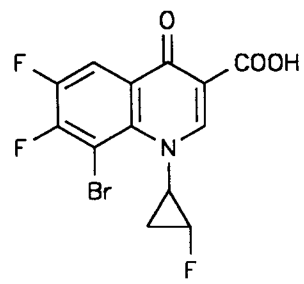

A. Zu einer Lösung von 7,6 g (20 mmol) 2-(3-Brom-2,4,5-trifluor-benzoyl)-3-ethoxy-acrylsäure-ethylester in 30 ml Ethanol werden 2,2 g (20 mmol) trans-2-Fluor-cyclopropylamin-Hydrochlorid und 1,2 g (10,7 mmol) 1,4-Diazabicyclo[2.2.2]octan hinzugefügt und die Mischung über Nacht bei Raumtemperatur gerührt. Der ausgefallene Niederschlag wird abgesaugt, mit 40 ml Wasser behandelt und getrocknet.
Ausbeute: 5,9 g (72 % der Theorie) 2-(3-Brom-2,4,5-trifluor-benzoyl)-3-(trans-2-fluor-cyclopropylamino)-acrylsäure-ethylester,
Schmelzpunkt: 99-100 °C.

B. 5,8 g (14 mmol)2-(3-Brom-2,4,5-trifluor-benzoyl)-3-(trans-2-fluor-cyclopropylamino)-acrylsäure-ethylester werden in 25 ml Dimethylformamid mit 1,1 g (26 mmol) Natriumfluorid 2 Stunden unter Rückfluß erhitzt. Die Suspension wird in 100 ml Eiswasser eingetragen, 30 Minuten verrührt und abgesaugt. Der Niederschlag wird mit Wasser gewaschen und bei 100 °C im Hochvakuum getrocknet.
Ausbeute: 5,1 g (92,7 % der Theorie) 8-Brom-6,7-difluor-1-(trans-2-fluor-cyclopropyl)-1,4-dihydro-4-oxo-3-chinolincarbonsäureethylester,
Schmelzpunkt: 172-174 °C (unter Zersetzung).

C. 5,1 g (13 mmol) 8-Brom-6,7-difluor-1-(trans-2-fluor-cyclopropyl)-1,4-dihydro-4-oxo-3-chinolincarbonsäureethylester werden in einer Mischung von 23 ml Essigsäure und 15 ml Wasser mit 2,5 ml konzentrierter Schwefelsäure versetzt. Man eerhitzt 2 Stunden unter Rückfluß, wobei die Mischung zunächst in Lösung geht und nach etwa 30 Minuten die Säure ausfällt. Man trägt die Mischung in 200 ml Eiswasser ein, saugt den Niederschlag ab, wäscht mit Wasser und trocknet bei 100 °C im Hochvakuum.
Ausbeute: 4,3 g (96 % der Theorie) 8-Brom-6,7-difluor-1-(trans-2-fluor-cyclopropyl)-1,4-dihydro-4-oxo-3-chinolincarbonsäure,
Schmelzpunkt: 224-225 °C (unter Zersetzung) (aus Glykolmonomethylether),
$^1$H-NMR (DMSO): $\delta$ 8,8 s (1 H), 8,3 ppm dd (1 H).

Analog wird 8-Brom-6,7-difluor-1-(cis-2-fluor-cyclopropyl)-1,4-dihydro-4-oxo-3-chinolincarbonsäure hergestellt.

Beispiel Z 3

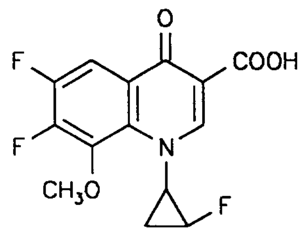

A. Zu einer Suspension von 613 mg (5,5 mmol) racemischem cis-2-Fluorcyclopropylamin-Hydrochlorid in 2,5 ml Essigsäure gibt man unter Eiskühlung 325 mg (2,9 mmol) 1,4-Diazabicyclo[2.2.2]-octan und tropft eine Lösung aus 1,66 g (5 mmol) 3-Ethoxy-2-(2,4,5-trifluor-3-methoxy-benzoyl)-acrylsäureethylester in 2 ml Essigsäure zu. Man läßt ohne Kühlung 4 Stunden nachrühren, erhitzt noch 1 Stunde auf 50 °C, engt die Suspension ein und verrührt den Rückstand mit etwa 15 ml Wasser. Der Niederschlag wird abgesaugt, mit Wasser gewaschen und getrocknet.
Ausbeute: 1,3 g (72 % der Theorie) 3-(cis-2-Fluor-cyclopropylamino)-2-(2,4,5-trifluor-3-methoxy-benzoyl)-acrylsäureethylester,
Schmelzpunkt: 82-83 °C.

B. 1,3 g (3,6 mmol) 3-(cis-2-Fluor-cyclopropylamino)-2-(2,4,5-trifluor-3-methoxy-benzoyl)-acrylsäureethylester werden in 30 ml wasserfreiem Tetrahydrofuran mit 160 mg Natriumhydrid (97 %ig) versetzt und 1 Stunde bei Raumtemperatur gerührt. Man verrührt mit 10 ml 1 n Salzsäure, konzentriert etwas auf und extrahiert mit ca. 50 ml Essigsäureethylester. Die organische Phase wird abgetrennt, mit Wasser gewaschen, mit Natriumsulfat getrocknet und eingeengt. Der schmierige Rückstand wird mit ca. 20 ml Diethylether verrührt und das ausgefallene Kristallisat abgesaugt und getrocknet.
Ausbeute: 814 mg (66 % der Theorie) 6,7-Difluor-1-(cis-2-fluor-cyclopropyl)-1,4-dihydro-8-methoxy-4-oxo-3-chinolincarbonsäureethylester,
Schmelzpunkt: 140-142 °C.

C. 610 mg (1,8 mmol) 6,7-Difluor-1-(cis-2-fluor-cyclopropyl)-1,4-dihydro-8-methoxy-4-oxo-3-chinolincarbonsäureethylester werden in einer Mischung aus 3,5 ml Essigsäure, 2,4 ml Wasser und 0,4 ml konzentrierter Schwefelsäure 2 Stunden bei 150 °C hydrolysiert. Die Suspension wird auf Eis gegossen, gut verrührt, der Niederschlag abgesaugt und mit Wasser und ca. 5 ml Methanol gewaschen und bei 60 °C im Hochvakuum getrocknet.
Ausbeute: 519 mg (93 % der Theorie) 6,7-Difluor-1-(cis-2-fluor-cyclopropyl)-1,4-dihydro-8-methoxy-4-oxo-3-chinolincarbonsäure,
Schmelzpunkt: 180-182 °C (unter Zersetzung), bleibt nach Umkristallisation aus Glykolmonomethylether unverändert.
$^1$H-NMR (DMSO): $\delta$ 8,8 s (1 H), 8,0 dd (1 H), 5,25 dm und 4,95 ppm dm (zusammen 1 H: CH-F).

Beispiel Z 4

Enantiomeren-Trennung von 6,7,8-Trifluor-1-(cis-2-fluor-cyclopropyl)-1,4-dihydro-4-oxo-3-chinolincarbonsäure: Eine Lösung von 1 g 6,7,8-Trifluor-1-(cis-2-fluor-cyclopropyl)-1,4-dihydro-4-oxo-3-chinolincarbonsäure in 100 g Tetrahydrofuran und 150 ml Toluol wird auf eine Chromatographiesäule (Betthöhe: 350 mm; Durchmesser: 120 mm) mit einem Perlpolymerisat von N-(Acryloyl-L-phenylalanin-D-menthylester (siehe Europäische Patentanmeldung 379 917) als stationärer Phase aufgegeben. Man eluiert mit Toluol/Tetrahydrofuran 5:1 (v:v) bei einem Fluß von 8 ml/min. Zur Detektion verwendet man ein Durchflußphotometer (Detektionswellenlänge 290 nm). Nach ca. 8 Stunden beginnt die Elution des ersten Enantiomeren. Die fraktionierten Eluate werden nach analytischer Kontrolle auf Enantiomerenreinheit vereinigt. Nach Abdampfen des Lösungsmittels erhält man jeweils 0,4 g beider Enantiomere.

Der Enantioselektivitätswert ($\alpha$-Wert) mit diesem Trennmaterial unter analytischen Bedingungen (Säule: 270 mm x 12,5 mm; Eluens: Toluol/Tetrahydrofuran (10/1 v/v); Fluß: 0,5 ml/min; Detektionswellenlänge: 290 nm) beträgt $\alpha$ = 2,16.

Beispiel Z 5

Analog Beispiel Z 4 können Enantiomeren-Trennungen auch an entsprechenden polymerbelegten Kieselgelphasen (siehe Europäische Patentanmeldung 379 917) durchgeführt werden: Säule: 250 mm x 4,6 mm; Eluens: n-Heptan/THF (3/2 v/v); Fluß: 1 ml/min; Detektionswellenlänge: 280 nm. Hierbei werden die folgenden Enantioselektivitätswerte (α-Werte) gemessen:
6,7,8-Trifluor-1-(cis-2-fluor-cyclopropyl)-1,4-dihydro-4-oxo-3-chinolincarbonsäure (α-Wert: 1,98),
6,7,8-Trifluor-1-(trans-2-fluor-cyclopropyl)-1,4-dihydro-4-oxo-3-chinolincarbonsäure (α-Wert: 1,00),
5,6,7,8-Tetrafluor-1-(trans-2-fluor-cyclopropyl)-1,4-dihydro-4-oxo-3-chinolincarbonsäure (α-Wert: 1,13),
8-Chlor-6,7-difluor-1-(cis-2-fluor-cyclopropyl)-1,4-dihydro-4-oxo-3-chinolincarbonsäure (α-Wert: 1,11),
8-Chlor-6,7-difluor-1-(trans-2-fluor-cyclopropyl)-1,4-dihydro-4-oxo-3-chinolincarbonsäure (α-Wert: 1,21),
6,7-Difluor-1-(cis-2-fluor-cyclopropyl)-1,4-dihydro-4-oxo-3-chinolincarbonsäure (α-Wert: 2,55),
6,7-Difluor-1-(trans-2-fluor-cyclopropyl)-1,4-dihydro-4-oxo-3-chinolincarbonsäure (α-Wert: 1,08),
7-Chlor-6-fluor-1-(cis-2-fluor-cyclopropyl)-1,4-dihydro-4-oxo-1,8-naphthyridin-3-carbonsäure (α-Wert: 2,28),
7-Chlor-6-fluor-1-(trans-2-fluor-cyclopropyl)-1,4-dihydro-4-oxo-1,8-naphthyridin-3-carbonsäure (α-Wert: 1,07),
8-Brom-6,7-difluor-1-(trans-2-fluor-cyclopropyl)-1,4-dihydro-4-oxo-3-chinolincarbonsäure (α-Wert: 1,11),
6,7-Difluor-1-(cis-2-fluor-cyclopropyl)-1,4-dihydro-8-methoxy-4-oxo-3-chinolincarbonsäure (α-Wert: 1,71).

## Herstellung der Wirkstoffe

Beispiel 1

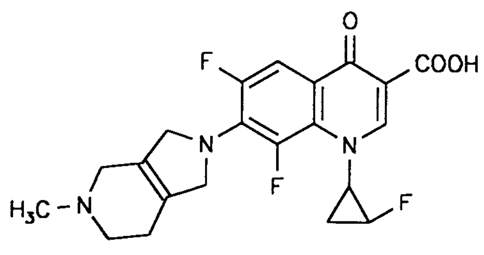

3,0 g (10 mmol) 6,7,8-Trifluor-1-(cis-2-fluorcyclopropyl)-1,4-dihydro-4-oxo-3-chinolincarbonsäure werden in einer Mischung aus 40 ml Acetonitril und 20 ml Dimethylformamid mit 1,25 g (11 mmol) 1,4-Diazabicyclo[2.2.2]octan und 1,65 g (11 mmol) 5-Methyl-2,3,4,5,6,7-hexahydro-1H-pyrrolo[3,4-c]pyridin 1 Stunde unter Rückfluß erhitzt. Die Suspension wird bei 70 ° C/20 mbar eingeengt und der Rückstand mit Wasser verrührt. Der Niederschlag wird abgesaugt, mit Wasser gewaschen und bei 100 ° C im Vakuum getrocknet.
Ausbeute: 3,7 g (88 % der Theorie) 6,8-Difluor-1-(cis-2-fluorcyclopropyl)-1,4-dihydro-7-(5-methyl-2,3,4,5,6,7-hexahydro-1H-pyrrolo[3,4-c]pyridin-2-yl)-4-oxo-3-chinolincarbonsäure;
Schmelzpunkt: 224-226 ° C (unter Zersetzung).
$^1$H-NMR ($CDCl_3$): δ um 8,68 ppm 2 s (1 H) (die Aufspaltung dieses Signals deutet auf 2 Rotamere).

Beispiel 2

Unter entsprechenden Bedingungen wie in Beispiel 1 erhält man mit (-)-6,7,8-Trifluor-1-(cis-2-fluorcyclopropyl)-1,4-dihydro-4-oxo-3-chinolincarbonsäure (-)-6,8-Difluor-1-(cis-2-fluorcyclopropyl)-1,4-dihydro-7-(5-methyl-2,3,4,5,6,7-hexahydro-1H-pyrrolo[3,4-c]pyridin-2-yl)-4-oxo-3-chinolincarbonsäure,
$[\alpha]_D^{26}$: -18 ° (c = 0,5, DMF),
Schmelzpunkt: 238-240 ° C (unter Zersetzung).

Beispiel 3

Unter entsprechenden Bedingungen wie in Beispiel 1 erhält man mit (+)-6,7,8-Trifluor-1-(cis-2-fluorcyclopropyl)-1,4-dihydro-4-oxo-3-chinolincarbonsäure (+)-6,8-Difluor-1-(cis-2-fluorcyclopropyl)-1,4-dihydro-7-(5-methyl-2,3,4,5,6,7-hexahydro-1H-pyrrolo[3,4-c]pyridin-2-yl)-4-oxo-3-chinolincarbonsäure,
$[\alpha]_D^{26}$: +18° (c = 0,5, DMF),
Schmelzpunkt: 240-242 °C (unter Zersetzung).

Beispiel 4

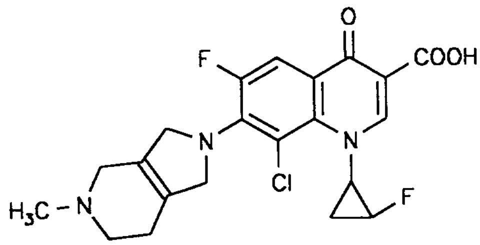

Analog Beispiel 1 wird mit 8-Chlor-6,7-difluor-1-(cis-2-fluorcyclopropyl)-1,4-dihydro-4-oxo-3-chinolincarbonsäure zu 8-Chlor-6-fluor-1-(cis-2-fluorcyclopropyl)-1,4-dihydro-7-(5-methyl-2,3,4,5,6,7-hexahydro-1H-pyrrolo[3,4-c]pyridin-2-yl)-4-oxo-3-chinolincarbonsäure vom Schmelzpunkt 204-206 °C (unter Zersetzung) umgesetzt.
$^1$H-NMR ($CF_3COOD$): δ um 9,3 ppm 2 s (1 H) (die Aufspaltung dieses Signals deutet auf 2 Rotamere).

Beispiel 5

Analog Beispiel 1 wird mit 6,7-Difluor-1-(cis-2-fluorcyclopropyl)-1,4-dihydro-4-oxo-3-chinolincarbonsäure zu 6-Fluor-1-(cis-2-fluorcyclopropyl)-1,4-dihydro-7-(5-methyl-2,3,4,5,6,7-hexahydro-1H-pyrrolo[3,4-c]pyridin-2-yl)-4-oxo-3-chinolincarbonsäure umgesetzt.

Beispiel 6

301 mg (1mmol) 6-Chlor-7-fluor-1-(cis-2-fluorcyclopropyl)-1,4-dihydro-4-oxo-1,8-naphthyridon-3-carbonsäure werden in 3 ml Acetonitril bei Raumtemperatur vorgelegt, mit 320 mg (2,1 mmol) 5-Methyl-2,3,4,5,6,7-hexahydro-1H-pyrrolo[3,4-c]pyridin versetzt und 6 Stunden bei Raumtemperatur gerührt. Man saugt ab, wäscht mit Acetonitril, nimmt in 4 ml 1n-Salzsäure in der Wärme auf und filtriert durch eine Fritte. Aus dem Filtrat isoliert man nach Konzentrieren und Behandeln mit Ethanol 6-Chlor-1-(cis-2-fluorcyclopropyl)-1,4-dihydro-7-(5-methyl-2,3,4,5,6,7-hexahydro-1H-pyrrolo[3,4-c]pyridin-2-yl)-4-oxo-1,8-naphthyridon-3-carbonsäure-Hydrochlorid.

Beispiel 7

Analog Beispiel 1 wird mit 8-Chlor-6,7-difluor-1-(trans-2-fluorcyclopropyl)-1,4-dihydro-4-oxo-3-chinolincarbonsäure zu 8-Chlor-6-fluor-1-(trans-2-fluorcyclopropyl)-1,4-dihydro-7-(5-methyl-2,3,4,5,6,7-hexahydro-1H-pyrrolo[3,4-c]pyridin-2-yl)-4-oxo-3-chinolincarbonsäure vom Schmelzpunkt 219-221 °C (unter Zersetzung) umgesetzt.

Beispiel 8

Analog Beispiel 1 wird mit 5,6,7,8-Tetrafluor-1-(trans-2-fluorcyclopropyl)-1,4-dihydro-4-oxo-3-chinolincarbonsäure zu 5,6,8-Trifluor-1-(trans-2-fluorcyclopropyl)-1,4-dihydro-7-(5-methyl-2,3,4,5,6,7-hexahydro-1H-pyrrolo[3,4-c]pyridin-2-yl)-4-oxo-3-chinolincarbonsäure umgesetzt.

Beispiel 9

Analog Beispiel 1 wird mit 8-Brom-6,7-difluor-1-(trans-2-fluorcyclopropyl)-1,4-dihydro-4-oxo-3-chinolincarbonsäure zu 8-Brom-6-fluor-1-(trans-2-fluorcyclopropyl)-1,4-dihydro-7-(5-methyl-2,3,4,5,6,7-hexahydro-1H-pyrrolo[3,4-c]pyridin-2-yl)-4-oxo-3-chinolincarbonsäure vom Schmelzpunkt 213-215 °C (unter Zersetzung) umgesetzt.

Beispiel 10

301 mg (1mmol) 7-Chlor-6-fluor-1-(cis-2-fluorcyclopropyl)-1,4-dihydro-4-oxo-1,8-naphthyridon-3-carbonsäure werden in 8 ml Acetonitril bei Raumtemperatur vorgelegt, mit 320 mg (2,1 mmol) 5-Methyl-2,3,4,5,6,7-hexahydro-1H-pyrrolo[3,4-c]pyridin versetzt und 6 Stunden bei Raumtemperatur gerührt. Man saugt ab, wäscht mit Acetonitril und erhält 296 mg (74 % der Theorie) 6-Fluor-1-(cis-2-fluorcyclopropyl)-1,4-dihydro-7-(5-methyl-2,3,4,5,6,7-hexahydro-1H-pyrrolo[3,4-c]pyridin-2-yl)-4-oxo-1,8-naphthyridon-3-carbonsäure vom Schmelzpunkt 254-257 °C (unter Zersetzung). Diese nimmt man in 4 ml 1n-Salzsäure in der Wärme auf und filtriert durch eine Fritte. Aus dem Filtrat isoliert man nach Konzentrieren und Behandeln mit Ethanol 6-Fluor-1-(cis-2-fluorcyclopropyl)-1,4-dihydro-7-(5-methyl-2,3,4,5,6,7-hexähydro-1H-pyrrolo[3,4-c]pyridin-2-yl)-4-oxo-1,8-naphthyridon-3-carbonsäure-Hydrochlorid.
Ausbeute: 219 mg (50 % der Theorie),
Schmelzpunkt: 321-325 °C (unter Zersetzung).

## Patentansprüche

**1.** Verbindungen der Formel (I)

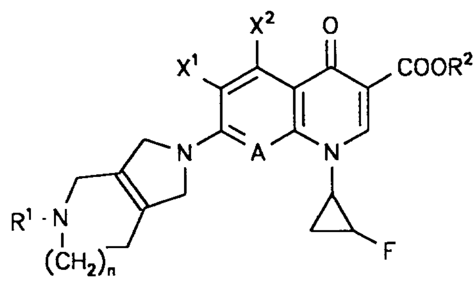

(I) ,

in welcher

- $R^1$ für Wasserstoff, gegebenenfalls durch Hydroxy substituiertes $C_1$-$C_3$-Alkyl, $C_1$-$C_4$-Alkoxycarbonyl, gegebenenfalls durch Halogen substituiertes Acetyl oder (5-Methyl-2-oxo-1,3-dioxol-4-yl)-methyl,
- $R^2$ für Wasserstoff, gegebenenfalls durch Hydroxy, Methoxy, Amino, Methylamino oder Dimethylamino substituiertes Alkyl mit 1 bis 3 Kohlenstoffatomen, (5-Methyl-2-oxo-1,3-dioxol-4-yl)-methyl, Acetoxymethyl oder Pivaloyloxymethyl,
- n für 0 oder 1,
- $X^1$ für Halogen oder Nitro,
- $X^2$ für Wasserstoff, Halogen, Amino oder Methyl,
- A für N, C-H, C-F, C-Cl, C-Br, $C\text{-}CF_3$, $C\text{-}OCH_3$, $C\text{-}OCHF_2$, $C\text{-}CH_3$ oder C-C≡CH steht

und deren pharmazeutisch verwendbaren Hydrate und Säureadditionssalze sowie die Alkali-, Erdalkali-, Silber- und Guanidiniumsalze der zugrundeliegenden Carbonsäuren.

**2.** Verbindungen der Formel (I) gemäß Anspruch 1,
in welcher

- $R^1$ für Wasserstoff, Methyl, Ethyl, t-Butoxycarbonyl, Acetyl, Trifluoracetyl oder Trichloracetyl
- $R^2$ für Wasserstoff,
- n für 0 oder 1,
- $X^1$ für Chlor oder Fluor,
- $X^2$ für Wasserstoff, Fluor, Amino oder Methyl,
- A für N, C-H, C-F, C-Cl, C-Br, $C\text{-}CF_3$, $C\text{-}OCH_3$, $C\text{-}OCHF_2$, $C\text{-}CH_3$ oder C-C≡CH steht

und deren pharmazeutisch verwendbaren Hydrate und Säureadditionssalze sowie die Alkali-, Erdalkali-, Silber- und Guanidiniumsalze der zugrundeliegenden Carbonsäuren.

**3.** Verbindungen der Formel (I) gemäß Anspruch 1,
in welcher

- $R^1$ für Wasserstoff oder Methyl,
- $R^2$ für Wasserstoff,

n für 1,
$X^1$ für Fluor,
$X^2$ für Wasserstoff, Fluor oder Amino,
A für N, C-H, C-F, C-Cl, C-Br, $C\text{-}OCH_3$ steht
und deren pharmazeutisch verwendbaren Hydrate und Säureadditionssalze sowie die Alkali-, Erdalkali-, Silber- und Guanidiniumsalze der zugrundeliegenden Carbonsäuren.

**4.** Enantiomerenreine Verbindungen nach den Ansprüchen 1 bis 3.

**5.** Verfahren zur Herstellung von Verbindungen der Formel (I) gemäß Ansprüchen 1 bis 4, dadurch gekennzeichnet, daß Verbindugnen der Formel (II)

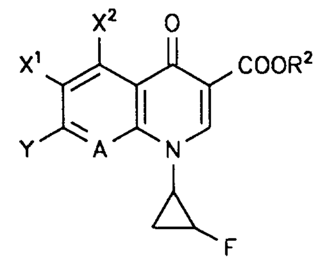

(II) ,

in welcher
$R^2$, $X^1$, $X^2$ und A die oben angegebene Bedeutung haben und
Y für Halogen, inbesondere für Fluor oder Chlor steht,
mit Verbindungen der Formel (III)

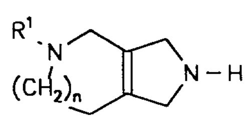

(III) ,

in welcher
$R^1$ und n die oben angegebenen Bedeutungen haben,
gegebenenfalls in Gegenwart von Säurefängern umgesetzt und gegebenenfalls vorhandene Schutzgruppen abgespalten werden.

**6.** Verfahren zur Herstellung von enantiomerenreinen Zwischenprodukten der Formel (II) gemäß Anspruch 5 dadurch gekennzeichnet, daß die entsprechenden Racemate an optisch aktiven Polymeren von optisch aktiven N-(Meth)-acryloyl-aminosäure-Derivaten mit Hilfe organischer Lösemittel chromatographisch getrennt werden.

**7.** Racemische und enantiomerenreine Verbindungen aus der Gruppe bestehend aus
5,6,7,8-Tetrafluor-1-(trans-2-fluor-cyclopropyl)-1,4-dihydro-4-oxo-3-chinolincarbonsäureethylester,
5,6,7,8-Tetrafluor-1-(trans-2-fluor-cyclopropyl)-1,4-dihydro-4-oxo-3-chinolincarbonsäure,
5,6,7,8-Tetrafluor-1-(cis-2-fluor-cyclopropyl)-1,4-dihydro-4-oxo-3-chinolincarbonsäureethylester,
5,6,7,8-Tetrafluor-1-(cis-2-fluor-cyclopropyl)-1,4-dihydro-4-oxo-3-chinolincarbonsäure,
8-Brom-6,7-difluor-1-(trans-2-fluor-cyclopropyl)-1,4-dihydro-4-oxo-3-chinolincarbonsäureethylester,
8-Brom-6,7-difluor-1-(trans-2-fluor-cyclopropyl)-1,4-dihydro-4-oxo-3-chinolincarbonsäure,
8-Brom-6,7-difluor-1-(cis-2-fluor-cyclopropyl)-1,4-dihydro-4-oxo-3-chinolincarbonsäureethylester,
8-Brom-6,7-difluor-1-(cis-2-fluor-cyclopropyl)-1,4-dihydro-4-oxo-3-chinolincarbonsäure,
6,7-Difluor-1-(trans-2-fluor-cyclopropyl)-1,4-dihydro-8-methoxy-4-oxo-3-chinolincarbonsäureethylester,
6,7-Difluor-1-(trans-2-fluor-cyclopropyl)-1,4-dihydro-8-methoxy-4-oxo-3-chinolincarbonsäure,
6,7-Difluor-1-(cis-2-fluor-cyclopropyl)-1,4-dihydro-8-methoxy-4-oxo-3-chinolincarbonsäureethylester,
6,7-Difluor-1-(cis-2-fluor-cyclopropyl)-1,4-dihydro-8-methoxy-4-oxo-3-chinolincarbonsäure.

**8.** Verbindungen gemäß Ansprüchen 1 bis 4 zur Bekämpfung von Krankheiten.

**9.** Verbindungen gemäß Ansprüchen 1 bis 4 zur Bekämpfung von bakteriellen Infektionen.

**10.** Verwendung von Verbindungen gemaß Ansprüchen 1 bis 4 für die Herstellung von Arzneimitteln.

**11.** Arzneimittel enthaltend die Verbindungen gemäß Ansprüchen 1 bis 4.

**12.** Antibakterielle Mittel enthaltend Verbindungen gemäß Ansprüchen 1 bis 4.

Europäisches Patentamt

# EUROPÄISCHER RECHERCHENBERICHT

Nummer der Anmeldung
EP 94 11 7335

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP-A-0 391 132 (BAYER AG) 10. Oktober 1990<br>* Seite 36 - Seite 43; Tabelle 1 *<br>* Beispiele 1,3 *<br>--- | 1-12 | C07D471/04<br>C07D487/04<br>C07D519/00<br>A61K31/47<br>//(C07D471/04, 221:00, 209:00), (C07D487/04, 209:00, 209:00), (C07D519/00, 471:00, 471:00), (C07D519/00, 487:00,471:00) |
| D,Y | EP-A-0 424 850 (KOREA RESEARCH INSTITUTE OF CHEMICAL TECHNOLOGY) 2. Mai 1991<br>* Beispiele 1-20,25-27 *<br>--- | 1-12 | |
| Y | EP-A-0 424 852 (KOREA RESEARCH INSTITUTE OF CHEMICAL TECHNOLOGY) 2. Mai 1991<br>* Seite 13, Zeile 20 - Seite 14, Zeile 10 *<br>* Seite 14, Zeile 26 - Seite 17, Zeile 30 *<br>--- | 1-12 | |
| D,Y | WO-A-92 21659 (DAIICHI PHARMACEUTICAL CO. LTD.) 10. Dezember 1992<br>* das ganze Dokument * | 1-12 | |
| P,Y | & EP-A-0 593 766 (...) 27. April 1994<br>--- | 1-12 | |
| D,Y | EP-A-0 520 277 (BAYER AG) 30. Dezember 1992<br>* Seite 6, Zeile 33 - Zeile 54 *<br>* Seite 14, Zeile 25 - Seite 15, Zeile 25 *<br>* Seite 15, Zeile 43 - Seite 16, Zeile 17 *<br>* Beispiele 7,8 *<br>--- | 1-12 | RECHERCHIERTE SACHGEBIETE (Int.Cl.6)<br>C07D<br>A61K |
| Y | EP-A-0 550 016 (KOREA RESEARCH INSTITUTE OF CHEMICAL TECHNOLOGY) 7. Juli 1993<br>* das ganze Dokument *<br>---<br>-/-- | 1-12 | |

Der vorliegende Recherchenbericht wurde für alle Patentansprüche erstellt

| Recherchenort | Abschlußdatum der Recherche | Prüfer |
|---|---|---|
| MÜNCHEN | 23. Dezember 1994 | Hartrampf, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung
EP 94 11 7335

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | JOURNAL OF MEDICINAL CHEMISTRY, Bd.36, Nr.22, 29. Oktober 1993 Seiten 3444 - 3448 ATARASHI S. ET AL. 'Fluorocyclopropyl quinolones. 1. Synthesis and structure-activity relationships of 1-(2-fluorocyclopropyl)-3-pyridonecarboxylic acid antibacterial agents' * das ganze Dokument * --- | 1-12 | |
| P,Y | EP-A-0 622 367 (KOREA RESEARCH INSTITUTE OF CHEMICAL TECHNOLOGY) 2. November 1994 * das ganze Dokument * ----- | 1-12 | |

RECHERCHIERTE SACHGEBIETE (Int.Cl.6)

Der vorliegende Recherchenbericht wurde für alle Patentansprüche erstellt

| Recherchenort | Abschlußdatum der Recherche | Prüfer |
|---|---|---|
| MÜNCHEN | 23. Dezember 1994 | Hartrampf, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)